(19) Europäisches Patentamt
European Patent Office
Office européen des brevets

(11) EP 1 078 883 B1

(12) **EUROPÄISCHE PATENTSCHRIFT**

(45) Veröffentlichungstag und Bekanntmachung des Hinweises auf die Patenterteilung:
**06.03.2002 Patentblatt 2002/10**

(51) Int Cl.[7]: **C01G 23/07**, C01G 23/08, C09C 1/36

(21) Anmeldenummer: **00112944.4**

(22) Anmeldetag: **20.06.2000**

# (54) Granulate auf Basis von pyrogen hergestelltem Titandioxid, Verfahren zu ihrer Herstellung und ihre Verwendung

Granules on the basis of pyrogenic titania, process for their preparation and use thereof

Granules à base de dioxyde de titane pyrogène, procédé pour leur préparation et leur utilisation

(84) Benannte Vertragsstaaten:
**AT BE CH CY DE DK ES FI FR GB GR IE IT LI LU MC NL PT SE**

(30) Priorität: **24.06.1999 DE 19928851**

(43) Veröffentlichungstag der Anmeldung:
**28.02.2001 Patentblatt 2001/09**

(73) Patentinhaber: **Degussa AG**
**40474 Düsseldorf (DE)**

(72) Erfinder:
- **Gilges, Hilmar, Dr.**
  **63739 Aschaffenburg (DE)**
- **Kerner, Dieter, Dr.**
  **63450 Hanau (DE)**
- **Meyer, Jürgen, Dr.**
  **63811 Stockstadt/Main (DE)**

(56) Entgegenhaltungen:
**EP-A- 0 241 647**
**EP-A- 0 722 992**
**EP-A- 0 725 037**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 078 883 B1

## Beschreibung

Die Erfindung betrifft Granulate auf Basis von pyrogen hergestelltem Titandioxid, das Verfahren zu ihrer Herstellung und ihre Verwendung.

Es ist bekannt, pyrogenes Titandioxid mittels Hochtemperatur oder Flammenhydrolyse aus $TiCl_4$ herzustellen (Ullmanns Enzyklopädie der technischen Chemie, 4. Auflage, Band 21, Seite 464 (1982)).

Pyrogene Titandioxide zeichnen sich durch extreme Feinteiligkeit, hohe spezifische Oberfläche (BET), sehr hohe Reinheit, sphärische Teilchenform und das Fehlen von Poren aus. Aufgrund dieser Eigenschaften finden pyrogen hergestellte Titandioxide zunehmend Interesse als Träger für Katalysatoren (Dr. Koth et al., Chem. Ing. Techn. 52, 628 (1980). Für diese Verwendung wird das pyrogen hergestellte Titandioxid auf mechanischem Wege mittels zum Beispiel Tablettiermaschinen verformt.

Es bestand somit die Aufgabe, Sprühgranulate von pyprogen hergestelltem Titandioxid, die als Katalysatorträger eingesetzt werden können, zu entwickeln.

Gegenstand der Erfindung sind Granulate auf Basis von pyrogen hergestelltem Titandioxid mit den folgenden physikalisch-chemischen Kenndaten:

| | |
|---|---|
| Mittlerer Korndurchmesser | 10 bis 150 µm |
| BET-Oberfläche | 25 bis 100 $m^2/g$ |
| pH-Wert | 3 bis 6 |
| Stampfdichte | 400 bis 1.200 g/l |

Das erfindungsgemäße Granulat kann hergestellt werden, indem man pyrogen hergestelltes Titandioxid in Wasser dispergiert und sprühtrocknet.

Ein weiterer Gegenstand der Erfindung sind Granulate auf Basis von pyrogen hergestelltem Titandioxid mit den folgenden physikalisch-chemischen Kenndaten:

| | |
|---|---|
| Mittlerer Korndurchmesser | 10 bis 160 µm |
| BET-Oberfläche | 15 bis 100 $m^2/g$ |
| pH-Wert | 3,0 bis 9,0 |
| Stampfdichte | 400 bis 1.200 g/l |
| Kohlenstoffgehalt | 0,3 bis 12,0 Gew.-% |

Das erfindungsgemäße Granulat kann hergestellt werden, indem man pyrogen hergestelltes Titandioxid in Wasser dispergiert, sprühtrocknet und anschliessend silanisiert. Zur Silanisierung können Halogensilane, Alkoxysilane, Silazane und/oder Siloxane eingesetzt werden.

Insbesondere können als Halogensilane die folgenden Stoffe eingesetzt werden:

Halogenorganosilane des Types $X_3Si(C_nH_{2n+1})$ — X = Cl, Br; n = 1 - 20

Halogenorganosilane des Types $X_2(R')Si(C_nH_{2n+1})$ — X = Cl, Br; R' = Alkyl; n = 1 - 20

Halogenorganosilane des Types $X(R')_2Si(C_nH_{2n+1})$ — X = Cl, Br; R' = Alkyl; n = 1 - 20

Halogenorganosilane des Types $X_3Si(CH_2)_m$-R'

X = Cl, Br
m = 0,1 - 20
R' = Alkyl, Aryl (z.B. $-C_6H_5$)
$-C_4F_9$, $-OCF_2-CHF-CF_3$, $-C_6F_{13}$, $-O-CF_2-CHF_2$

$-NH_2$, $-N_3$, -SCN, $-CH=CH_2$,
$-OOC(CH_3)C = CH_2$
$-OCH_2-CH(O)CH_2$
$-NH-CO-N-CO-(CH_2)_5$
$-NH-COO-CH_3$, $-NH-COO-CH_2-CH_3$, $-NH-(CH_2)_3Si(OR)_3$
$-S_x-(CH_2)_3Si(OR)_3$

Halogenorganosilane des Types $(R)X_2Si(CH_2)_m-R'$

X = Cl, Br
R = Alkyl
m = 0,1 - 20
R' = Alkyl, Aryl (z.B. $-C_6H_5$)
$-C_4F_9$, $-OCF_2-CHF-CF_3$, $-C_6F_{13}$, $-O-CF_2-CHF_2$
$-NH_2$, $-N_3$, -SCN, $-CH=CH_2$,
$-OOC(CH_3)C = CH_2$
$-OCH_2-CH(O)CH_2$
$-NH-CO-N-CO-(CH_2)_5$
$-NH-COO-CH_3$, $-NH-COO-CH_2-CH_3$, $-NH-(CH_2)_3Si(OR)_3$
$-S_x-(CH_2)_3Si(OR)_3$

Halogenorganosilane des Types $(R)_2X\ Si(CH_2)_m-R'$

X = Cl, Br
R = Alkyl
m = 0,1 - 20
R' = Alkyl, Aryl (z.B. $-C_6H_5$)
$-C_4F_9$, $-OCF_2-CHF-CF_3$, $-C_6F_{13}$, $-O-CF_2-CHF_2$
$-NH_2$, $-N_3$, -SCN, $-CH=CH_2$,
$-OOC(CH_3)C = CH_2$
$-OCH_2-CH(O)CH_2$
$-NH-CO-N-CO-(CH_2)_5$
$-NH-COO-CH_3$, $-NH-COO-CH_2-CH_3$, $-NH-(CH_2)_3Si(OR)_3$
$-S_x-(CH_2)_3Si(OR)_3$

Insbesondere können als Alkoxysilane die folgenden Stoffe eingesetzt werden:

Organosilane des Types $(RO)_3Si(C_nH_{2n+1})$
R = Alkyl
n = 1 - 20

Organosilane des Types $R'_x(RO)_ySi(C_nH_{2n+1})$
R = Alkyl
R' = Alkyl
n = 1 - 20
x+y = 3
x = 1,2
y = 1, 2

Organosilane des Types $(RO)_3Si(CH_2)_m-R'$

R = Alkyl
m = 0,1 - 20
R' = Alkyl, Aryl (z.B. $-C_6H_5$)
$-C_4F_9$, $OCF_2-CHF-CF_3$, $-C_6F_{13}$, $-O-CF_2-CHF_2$
$-NH_2$, $-N_3$, -SCN, $-CH=CH_2$,
$-OOC(CH_3)C = CH_2$
$-OCH_2-CH(O)CH_2$
$-NH-CO-N-CO-(CH_2)_5$
$-NH-COO-CH_3$, $-NH-COO-CH_2-CH_3$, $-NH-(CH_2)_3Si(OR)_3$

$-S_x-(CH_2)_3Si(OR)_3$

Organosilane des Typs $(R'')_x(RO)_ySi(CH_2)_m-R'$

R'' = Alkyl $x+y = 3$
- $x = 1,2$
- $y = 1,2$

R' = Alkyl, Aryl (z.B. $-C_6H_5$)
$-C_4F_9$, $-OCF_2-CHF-CF_3$, $-C_6F_{13}$, $-O-CF_2-CHF_2$
$-NH_2$, $-N_3$, -SCN, $-CH=CH_2$,
$-OOC(CH_3)C = CH_2$
$-OCH_2-CH(O)CH_2$
$-NH-CO-N-CO-(CH_2)_5$
$-NH-COO-CH_3$, $-NH-COO-CH_2-CH_3$, $-NH-(CH_2)_3Si(OR)_3$
$-S_x-(CH_2)_3Si(OR)_3$

Bevorzugt kann man als Silanisierungsmittel das Silan Si 108 [$(CH_3O)_3-Si-C_8H_{17}$] Trimethoxyoctylsilan einsetzen.

Insbesondere können als Silazane die folgenden Stoffe eingesetzt werden:

Silazane des Types

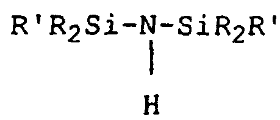

R = Alkyl
R' = Alkyl, Vinyl

sowie zum Beispiel Hexamethyldisilazan.

Insbesondere können als Siloxane die folgenden Stoffe eingesetzt werden:

Cyclische Polysiloxane des Types D 3, D 4, D 5
z.B. Octamethylcyclotetrasiloxan = D 4

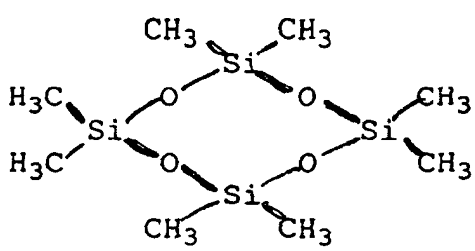

Polysiloxane bzw. Silikonöle des Types

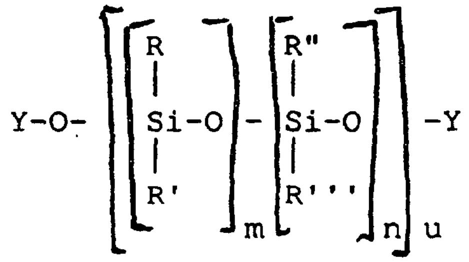

$m = 0,1,2,3,...\infty$

$n = 0,1,2,3,...\infty$

$u = 0,1,2,3,...\infty$

$Y = CH_3$, H, $C_nH_{2n+1}$ $n = 1-20$

$Y = Si(CH_3)_3$, $Si(CH_3)_2H$

$Si(CH_3)_2OH$, $Si(CH_3)_2(OCH_3)$

$Si(CH_3)_2(C_nH_{2n+1})$ $n = 1-20$

R = Alkyl, Aryl, $(CH_2)_n - NH_2$, H
R' = Alkyl, Aryl, $(CH_2)_n - NH_2$, H
R" = Alkyl, Aryl, $(CH_2)_n - NH_2$, H
R"' = Alkyl, Aryl, $(CH_2)_n - NH_2$, H

Der Kohlenstoffgehalt des erfindungsgemäßen Granulates kann 0,3 bis 12,0 Gew.-% betragen.

Die Dispersion in Wasser kann eine Konzentration an Titandioxid von 3 bis 25 Gew.-% aufweisen.

Zur Dispersion können organische Hilfsmittel zugefügt werden, um die Stabilität der Dispersion zu erhöhen und die Teilchenmorphologie nach der Sprühtrocknung zu verbessern.

Beispielsweise können die folgenden Hilfsstoffe eingesetzt werden:
Polyalkohole, Polyether, Tenside auf Fluorkohlenwasserstoffbasis, Alkohole.

Die Sprühtrocknung kann man bei einer Temperatur von 200 bis 600 °C durchführen. Dabei kann man Scheibenzerstäuber oder Düsenzerstäuber einsetzen.

Die Silanisierung kann mit denselben Halogensilanen, Alkoxysilanen, Silazanen und/oder Siloxanen wie oben beschrieben durchgeführt werden, wobei das Silanisierungsmittel gegebenenfalls in einem organischen Lösungsmittel, wie zum Beispiel Ethanol, gelöst sein kann.

Bevorzugt kann man als Silanisierungsmittel das Silan Si 108 [$(CH_3O)_3$-Si-$C_8H_{17}$] Trimethoxyoctylsilan einsetzen.

Die Silanisierung kann man durchführen, indem man das Granulat mit dem Silanisierungsmittel bei Raumtemperatur besprüht und das Gemisch anschließend bei einer Temperatur von 105 bis 400 °C über einen Zeitraum von 1 bis 6 h thermisch behandelt.

Eine alternative Methode der Silanisierung der Granulate kann man durchführen, indem man das Granulat mit dem Silanisierungsmittel in Dampfform behandelt und das Gemisch anschließend bei einer Temperatur von 200 bis 800 °C über einen Zeitraum von 0,5 bis 6 h thermisch behandelt.

Die thermische Behandlung kann unter Schutzgas, wie zum Beispiel Stickstoff, erfolgen.

Die Silanisierung kann man in beheizbaren Mischern und Trocknern mit Sprüheinrichtungen kontinuierlich oder ansatzweise durchführen. Geeignete Vorrichtungen können zum Beispiel sein: Pflugscharmischer, Teller-, Wirbelschicht- oder Fließbetttrockner.

Durch die Variation der Einsatzstoffe, der Bedingungen bei der Sprühung, der Temperung und der Silanisierung kann man die physikalisch-chemischen Parameter der Granulate, wie die spezifische Oberfläche, die Korngrößenverteilung, die Stampfdichte, und pH-Wert innerhalb der angegebenen Grenzen verändern.

Das erfindungsgemäße Titandioxidgranulat weist die folgenden Vorteile auf:

Das Fließverhalten ist besser als bei nichtsprühgetrocknetem Titandioxid.
Das Einarbeiten in organische Systeme ist leichter.
Die Dispergierung ist einfacher.
Für die Granulation sind keine zusätzlichen Hilfsstoffe erforderlich.
Gegenüber dem nichtsprühgertocknetem Titandioxid, das keine definierte Agglomeratgröße aufweist, hat das erfindungsgemäße Titandioxidgranulat eine definierte Teilchengröße.
Das erfindungsgemäße Titandioxidgranulat ermöglicht eine staubfreie Handhabung.
Auf Grund der hohen Stampfdichte ist ein geringerer Verpackungsaufwand für den Transport erforderlich.
Das erfindungsgemäße Titandioxidgranulat kann als Katalysatorträger eingesetzt werden.
Das nichtsprühgetrocknete Titandioxid ist hierzu nicht geeignet, weil es beispielsweise aus dem Wirbelbett herangetragen wird.

Die erfindungsgemäßen Granulate können als Träger für Katalysatoren, sowie in Kosmetika, als Sonnenschutz, in Silikonkautschuk, in Tonerpulver, in Lacken und Farben, als Schleif- und Poliermittel, als Rohstoff zur Glas- und Keramikherstellung eingesetzt werden.

**Beispiele**

Als pyrogen hergestelltes Titandioxid wird ein Titandioxid P25 mit den folgenden physikalisch-chemischen Kenndaten eingesetzt. Es ist bekannt aus der Schriftenreihe Pigmente Nr. 56 "Hochdisperse Metalloxide nach dem Aerosilverfahren", 4. Auflage, Februar 1989, Degussa AG.

| | **Titandioxid P 25** |
|---|---|
| CAS-Reg. Nummer | 13463-67-7 |
| Verhalten gegenüber Wasser | hydrophil |
| Aussehen | lockeres weißes Pulver |
| Oberfläche nach BET[1]) $m^2/g$ | 50 ± 15 |
| Mittlere Größe der Primärteilchen nm | 21 |
| Stampfdichte[2]) g/l | ca. 100 |
| Spezifisches Gewicht[10]) g/ml | ca. 3,7 |
| Trocknungsverlust[3]) bei Verlassen des Lieferwerkes % (2Stunden bei 105°C) | < 1,5 |
| Glühverlust[4]) [7]) (2 Stunden bei 1000°C) | < 2 |
| pH-Wert[5]) (in 4%iger wäßriger Dispersion) | 3-4 |
| $SiO_2$[8)] | < 0,2 |
| $Al_2O_3$[8)] | < 0,3 |
| $Fe_2O_3$[8)] | < 0,01 |
| $TiO_2$[8)] | > 99,5 |
| ZrO2[8)] | - |
| HfO2[8)] | - |
| HCl[8)9)] | < 0,3 |
| Siebrückstand[6)] (nach Mocker, 45 µm) % | < 0,05 |

1) nach DIN 66131
2) nach DIN ISO 787/XI, JIS K 5101/18 (nicht gesiebt)
3) nach DIN ISO 787/II, ASTM D 280, JIS K 5101/21
4) nach DIN 55921, ASTM D 1208, JIS K 5101/23
5) nach DIN ISO 787/IX;ASTM D !"=(; JIS K 5101/24
6) nach DIN ISO 787/XVIII; JIS K 5101/20
7) bezogen auf die 2 Stunden bei 105°C getrocknete Substanz
8) bezogen auf die 2 Stunden bei 1000°C geglühte Substanz
9)HCl-Gehalt ist Bestandteil des Glühverlustes
10) bestimmt mit dem Luftvergleichspyknometer

Zur Herstellung der Titandioxide wird in eine Knallgasflamme aus Wasserstoff und Luft eine flüchtige Titanverbindung eingedüst. In den meisten Fällen verwendet man Titantetrachlorid. Diese Substanz hydrolysiert unter dem Einfluß des bei der Knallgasreaktion entstehenden Wassers zu Titandioxid und Salzsäure. Das Titandioxid tritt nach dem Verlassen der Flamme in eine sogenannte Koagulationszone ein, in der die Titandioxid-Primärteilchen und -Primäraggregate agglomerieren. Das in diesem Stadium als eine Art Aerosol vorliegende Produkt wird in Zyklonen von den gasförmigen Begleitsubstanzen getrennt und anschließend mit feuchter Heißluft nachbehandelt.
Die Teilchengrößen der Titandioxide können mit Hilfe der Reaktionsbedingungen, wie zum Beispiel Flammentemperatur, Wasserstoff- oder Sauerstoffanteil, Titantetrachloridmenge, Verweilzeit in der Flamme oder Länge der Koagulationsstrecke, variiert werden.
Die BET-Oberfläche wird gemäß DIN 66 131 mit Stickstoff bestimmt.
Das Stampfvolumen wird in Anlehnung an ASTM D 4164-88 bestimmt.

Geräte: Stampfvolumeter STA V 2003 der Fa. Engelsmann nach DIN 53194, Abs. 5.2. b-f
Meßzylinder 250 ml, Teilstriche je 2 ml
Waage mit Fehlergrenze max. ± 0,1 g

Durchführung

Stelle das Zählerwerk des Stampfvolumeters auf 1000 Hübe.
Tariere den Meßzylinder.
Fülle Granulat in den Meßzylinder bis zu 250 ml Marke.
Notiere die Einwaage (± 0,1 g).
Spanne den Meßzylinder in das Stampfvolumeter und schalte das Gerät ein.
Stampfende → Gerät schaltet nach 1000 Hüben automatisch ab.
Lese das gestampfte Schüttvolumen auf 1 ml genau ab.

Berechnung

E: Granulateinwaage in g
V: Abgelesenes Volumen in ml
W: Wassergehalt in Gew.-% (bestimmt nach Prüfvorschrift P001)

$$\text{Stampfdichte} = \frac{E \times (100 - W)}{V \times 100}$$

Der pH-Wert wird in 4 %iger wäßriger Dispersion bestimmt, bei hydrophoben Katalysatorträgern in Wasser: Ethanol 1 : 1.

Herstellung der erfindungsgemäßen Granulate

Das pyrogen hergestellte Titandioxid wird in vollentsalztem Wasser dispergiert. Dabei wird ein Dispergieraggregat verwendet, das nach dem Rotor/Stator-Prinzip arbeitet. Die entstehenden Dispersionen werden sprühgetrocknet. Die Abscheidung des Fertigproduktes erfolgt über Filter oder Zyklon.
Die sprühgetrockneten Granulate werden zur Silanisierung in einem Mischer vorgelegt und unter intensivem Mischen gegebenenfalls zunächst mit Wasser und anschließend mit dem Silanisierungsmittel besprüht. Nachdem das Sprühen beendet ist, wird noch 15 bis 30 min nachgemischt und anschließend 1 bis 4 h bei 100 bis 400 °C getempert.
Das eingesetzte Wasser kann mit einer Säure, zum Beispiel Salzsäure, bis zu einem pH-Wert von 7 bis 1 angesäuert sein. Das eingesetzte Silanisierungsmittel kann in einem Lösungsmittel, wie zum Beispiel Ethanol, gelöst sein.

Tabelle 1

Daten zur Sprühtrocknung von wässrigen $TiO_2$P25-Dispersionen

| Beispiel | Menge $H_2O$ [kg] | Menge $TiO_2$P25 [kg] | Zerstäubung mit | Dehzahl Zerstäuberscheibe [Upm] | Betriebstemperatur [°C] | Ablufttemperatur [°C] | Abscheidung |
|---|---|---|---|---|---|---|---|
| 1 | 10 | 1,5 | Scheibe | 35 000 | 345 | 100 | Zyklon |
| 2 | 10 | 1,5 | Scheibe | 45 000 | 370 | 105 | Zyklon |
| 3 | 10 | 1,5 | Scheibe | 20 000 | 350 | 95 | Zyklon |
| 4 | 10 | 2,5 | Scheibe | 15 000 | 348 | 100 | Zyklon |
| 5 | 100 | 15 | 2-Stoff-Düse | --- | 445 | 130 | Filter |
| 6 | 100 | 15 | Scheibe | 10 000 | 450 | 105 | Filter |
| 7 | 10 | 2,5 | Scheibe | 20 000 | 348 | 105 | Zyklon |
| 8 | 10 | 1,5 | Scheibe | 15 000 | 348 | 105 | Zyklon |
| 9 | 10 | 2,5 | Scheibe | 35 000 | 300 | 105 | Zyklon |

Tabelle 2:

Physikalisch-chemische Daten der sprühgetrokneten Produkte

| **Beispiel** | **Oberfläche nach BET [$m^2/g$]** | **Stampfdichte [g/l]** | **pH-Wert** | **$d_{50}$-Wert (Cilas) [µm]** | **Trocknungs -verlust [%]** | **Glühverlust [%]** |
|---|---|---|---|---|---|---|
| 1 | 51 | 641 | 3,9 | 14,6 | 0,9 | 0,9 |
| 2 | 50 | 612 | 3,7 | 10,6 | 0,8 | 1,0 |
| 3 | 52 | 680 | 3,5 | 25,0 | 0,8 | 1,0 |
| 4 | 51 | 710 | 3,7 | 43,6 | 0,8 | 1,2 |
| 5 | 52 | 660 | 4,0 | 17,1 | 0,9 | 0,9 |
| 6 | 53 | 702 | 3,9 | 27,5 | 0,9 | 0,9 |
| 7 | 50 | 708 | 3,5 | 26,7 | 1,1 | 0,6 |
| 8 | 53 | 696 | 3,9 | 30,1 | 1,0 | 0,9 |
| 9 | 49 | 640 | 3,7 | 16,0 | 0,7 | 0,8 |

Tabelle 3:

Daten zur Hydrophobierung von dem Titandioxidgranulat gemäß Beispiel 6

| Beispiel | Menge P25/7 [kg] | Hydrophobierungs-mittel | Menge Hydrophobierungs-mittel [kg] | Menge $H_2O$ [kg] | Temper Temperatur [°C] | Temper-zeit [Stdn] |
|---|---|---|---|---|---|---|
| 10 | 2 | Si 108 | 0,2 | 0,05 | 120 | 2 |
| 11 | 2 | Si 108 | 0,3 | 0,05 | 120 | 2 |
| 12 | 3,15 | Silikonöl | 0,2 | 0 | 350 | 2 |
| 13 | 3,15 | Silikonöl | 0,3 | 0 | 350 | 15 |

Tabelle 4:

Physikalisch-chemische Daten von hydrophobiertem Titandioxidgranulat gemäß Beispiel 6

| Beispiel | Oberfläche nach BET [$m^2/g$] | C-Gehalt [%] | Stampfdichte [g/l] | pH-Wert | Trocknungs-verlust [%] | Glühverlust [%] | $d_{50}$-Wert (Cilas) [µm] |
|---|---|---|---|---|---|---|---|
| 10 | 36 | 3,9 | 848 | 3,1 | 0,2 | 4,2 | 29,8 |
| 11 | 30 | 5,5 | 873 | 3,2 | 0,4 | 6,1 | 28,7 |
| 12 | 32 | 2,1 | 768 | 3,6 | 0 | 1,9 | 30,2 |
| 13 | 25 | 3,3 | 883 | 3,9 | 0 | 4,4 | 28,1 |

**Patentansprüche**

1. Granulate auf Basis von pyrogen hergestelltem Titandioxid mit den folgenden physikalisch-chemischen Kenndaten:

| | |
|---|---|
| Mittlerer Korndurchmesser | 10 bis 150 µm |
| BET-Oberfläche | 25 bis 100 $m^2/g$ |
| pH-Wert | 3 bis 6 |
| Stampfdichte (nach ASTM D 4164-88) | 400 bis 1.200 g/l |

2. Verfahren zur Herstellung der Granulate nach Anspruch 1, **dadurch gekennzeichnet, daß** man pyrogen hergestelltes Titandioxid in Wasser dispergiert, sprühtrocknet.

3. Granulate auf Basis von pyrogen hergestelltem Titandioxid mit den folgenden physikalisch-chemischen Kenndaten:

| | |
|---|---|
| Mittlerer Korndurchmesser | 10 bis 160 µm |
| BET-Oberfläche | 15 bis 100 $m^2/g$ |
| pH-Wert | 3,0 bis 9,0 |
| Stampfdichte (nach ASTM D 4164-88) | 400 bis 1.200 g/l |
| Kohlenstoffgehalt | 0,3 bis 12,0 Gew.-% |

4. Verfahren zur Herstellung der Granulate nach Anspruch 3, **dadurch gekennzeichnet, daß** man pyrogen hergestelltes Titandioxid in Wasser dispergiert, sprühtrocknet und anschließend silanisiert.

5. Verwendung der Granulate gemäß den Ansprüchen 1 und 3 als Katalysatorträger, sowie in Kosmetika, als Sonnenschutz, in Silikonkautschuk, in Tonerpulver, in Lacken und Farbe, als Schleif- und Poliermittel, als Rohstoff für Glas- und Keramikherstellung.

**Claims**

1. Granules based on pyrogenically produced titanium dioxide and having the following physicochemical properties:

| | |
|---|---|
| Average grain size | 10 to 150 µm |
| BET surface area | 25 to 100 $m^2/g$ |
| pH value | 3 to 6 |
| Tamped density (as in ASTM D 4164-88) | 400 to 1,200 g/l. |

2. Process for producing the granules according to claim 1, **characterised in that** pyrogenically produced titanium dioxide is dispersed in water and spray dried.

3. Granules based on pyrogenically produced titanium dioxide and having the following physicochemical properties:

| | |
|---|---|
| Average grain size | 10 to 160 µm |
| BET surface area | 15 to 100 $m^2/g$ |
| pH value | 3.0 to 9.0 |
| Tamped density (as in ASTM D 4164-88) | 400 to 1,200 g/l |
| Carbon content | 0.3 to 12.0 wt.%. |

4. Process for producing the granules according to claim 3, **characterised in that** pyrogenically produced titanium dioxide is dispersed in water, spray dried and then silanised.

**5.** Use of the granules according to claims 1 and 3 as catalyst supports, as well as in cosmetics, as sunscreen, in silicone rubber, in toner powder, in varnishes and paints, as abrasives and polishes, as raw material for the production of glass and ceramic.

## Revendications

**1.** Produits granulés à base de dioxyde de titane préparé par pyrogénation, ayant les caractéristiques physico-chimiques suivantes :

| | |
|---|---|
| Diamètre moyen de grain | 10 à 150 µm |
| Surface spécifique BET | 25 à 100 $m^2/g$ |
| pH | 3 à 6 |
| Densité apparente à l'état tassé, (selon ASTM D 4164-88) | 400 à 1 200 g/l. |

**2.** Procédé pour la préparation des produits granulés selon la revendication 1,
**caractérisé en ce qu'**
on disperse dans de l'eau du dioxyde de titane préparé par pyrogénation, et on le sèche par atomisation.

**3.** Produits granulés à base de dioxyde de titane préparé par pyrogénation, ayant les caractéristiques physico-chimiques suivantes :

| | |
|---|---|
| Diamètre moyen de grain | 10 à 160 µm |
| Surface spécifique BET | 15 à 100 $m^2/g$ |
| pH | 3,0 à 9,0 |
| Densité apparente à l'état tassé (selon ASTM D 4164-88) | 400 à 1 200 g/l |
| Teneur en carbone | 0,3 à 12,0 % en poids. |

**4.** Procédé pour la préparation des produits granulés selon la revendication 3,
**caractérisé en ce qu'**
on disperse dans de l'eau du dioxyde de titane préparé par pyrogénation, on le sèche par atomisation et ensuite on le, soumet à une silanation.

**5.** Utilisation des produits granulés selon les revendications 1 et 3, en tant que support de catalyseur, ainsi que dans des cosmétiques, en tant qu'écran solaire, dans des caoutchoucs silicone, dans des poudres de toner, dans des peintures et des encres, en tant qu'agent de polissage et abrasif, en tant que matière première pour la fabrication de verre et de céramiques.